# EUROPEAN PATENT SPECIFICATION

(11) **EP 1 420 842 B1**
(45) Date of publication and mention of the grant of the patent: **08.11.2006**
(21) Application number: 02760976.7
(22) Date of filing: 26.08.2002
(51) Int. Cl.: A61M 16/00

(54) **DEVICE FOR QUANTITATIVE ANALYSIS OF RESPIRATORY GASES**
VORRICHTUNG ZUR QUANTITATIVEN ANALYSE VON ATEMGASEN
DISPOSITIF DESTINE A L'ANALYSE QUANTITATIVE DE GAZ RESPIRATOIRES

(30) Priority: 28.08.2001 SE 0102861
(43) Date of publication of application: 26.05.2004
(73) Proprietor: Phase-In AB, 111 73 Stockholm (SE)
(72) Inventor: ECKERBOM, Anders, S-185 41 VAXHOLM (SE)
(74) Representative: Karlström, Lennart
(86) International application number: PCT/SE2002/001527
(87) International publication number: WO 2003/018093

(56) References cited:
- GB-A- 2 287 655
- US-A- 5 701 888
- US-A- 6 003 511

## Description

The present invention relates to an arrangement pertaining to the quantitative analysis of respiratory gases to and from a patient connected to a respirator for breathing assistance.

With regard to gas analysis carried out in connection with respiratory care, a distinction is made between two principle types of gas analysers, i.e. between lateral flow measuring analysers and main flow measuring analysers. The lateral flow measuring analysers take a minor sample flow from the respiratory circuit of a patient to an adjacent instrument in which the actual gas analysis takes,place, whereas the main flow measuring analysers calculate the gas concentrations directly in the respiratory circuit of the patient. The main flow measuring analyser is normally placed as close as possible to the patient's mouth or trachea, for reasons of accuracy.

The main flow measuring analysers can be made less expensive, smaller, more energy-lean and more responsive than the lateral flow measuring analysers, since the need for sample flow handling (pumps, hoses, etc.) is obviated. Consequently, the main flow measuring gas analysers are preferred over the lateral flow measuring analysers.

Various requirements for gas analyses exist in health care. For example, it is sufficient to monitor breathing of a patient with a simple carbon dioxide analysis in the case of emergency care, whereas it is often desired to measure and monitor a greater number of patient gases, such as carbon dioxide, oxygen gas, nitrous oxide and one or more of the anaesthesia agents Halothan, Enfluran, Isofluran, Sevofluran and Desfluran in the case of patient anaesthesia.

For reasons of a technical nature, it has been difficult to develop main flow measuring patient-gas analysers other than for carbon dioxide. Although such analysers have found a broad use spectrum in emergency care in particular, the use of lateral flow measuring analysers has been referred to in other care aspects, such as intensive care and anaesthesia, for instance, due to the technical problems that occur, primarily related to moisture and bacteria in the patient's respiratory circuit.

Respiratory gases can be analysed in accordance with different measuring principles. The most common method of gas analysis, however, is through the medium of non-dispersive spectroscopy. This measuring principle is based on the fact that many gases absorb infrared energy at a wavelength specific for the substance concerned. Main flow measuring gas analysers based on non-dispersive spectroscopy measure light absorption at specific wavelengths directly in the patient's respiratory circuit. An earlier known design of one such gas analyser is described in WO91/18279 A1, for instance. In the case of this gas analyser, a broadband infrared light beam is allowed to pass through the patient's respiratory circuit. The light beam is then divided by a beam splitter into two beams, which are registered by two separate detectors provided with optical bandpass filters having mutually different centre wavelengths. One detector is used to calculate the intensity of the light beam at the absorption wavelength of the analysis substance, whereas the other detector is used to calculate a measurement of the reference intensity of the light beam at a wavelength different from the absorption wavelength of the analysis substance. This type of gas analyser is well suited for the analysis of individual gases, such as carbon dioxide, for instance. However, intensity losses in the beam splitter and the size of the beam splitter make this type of analyser unsuitable for the multigas analysis based on main flow.

Unfortunately, oxygen gas exhibits no marked absorption within the infrared range and, in respect of oxygen gas analysis, there are normally used fuel cells or analysers that utilise the paramagnetic properties of oxygen gas. These latter solutions are highly shock sensitive, which makes them unsuitable for main flow measuring analysis.

Fuel cells are comprised of a gold cathode and a lead cathode surrounded by an electrolyte protected by a membrane through which oxygen-gas defuses into the cell. The current generated by the cell is directly proportional to the partial pressure of the oxygen gas. The response time of the cell is dependent on the design of the membrane and its thickness, and also to the extent to which the gas yield is permitted to take place nearest the membrane. However, response times are normally in the magnitude of from one to ten seconds. Response times of such long duration have made it difficult to use fuel cells for registration of oxygen gas that is dissolved during main flow measuring gas analysis.

In GB-A-2 287 655 figure 3 a part of a fuel-cell is placed between a mouthpiece for a patient and a respirator , the rest of the fuel-cell is connected via a cable.

US-A-6 003 511 shows the use of a bacterial filter in front of unspecified functional devices.

Accordingly, an object of the present invention is to provide a novel arrangement with which the aforesaid problems can be avoided and which enables fuel cells to be used for measuring oxygen gas contents also in main flow measuring gas analysers.

The aforesaid object of the present invention is achieved with a gas analyser that includes an adapter having connectors for connection to a respirator or the like, and connectors for connection to a hose leading to the patient, wherein the adapter includes a fuel cell and a gas analyser measuring head between the respirator connector and the connectors for connecting hoses to the patient, and wherein the adapter is also provided with a bacteria filter for protecting the fuel cell from bacteria in the respiratory gases.

The invention will now be described in more detail with reference to a non-limiting embodiment thereof and also with reference to the accompanying drawings, in which **Fig. 1** is a schematic perspective view of an inventive arrangement with associated measuring head; **Fig. 2** is a schematic illustration of a patient connected to a respirator with the aid of the inventive arrangement; and **Fig. 3** is a schematic sectional view of an adapter according to the invention.

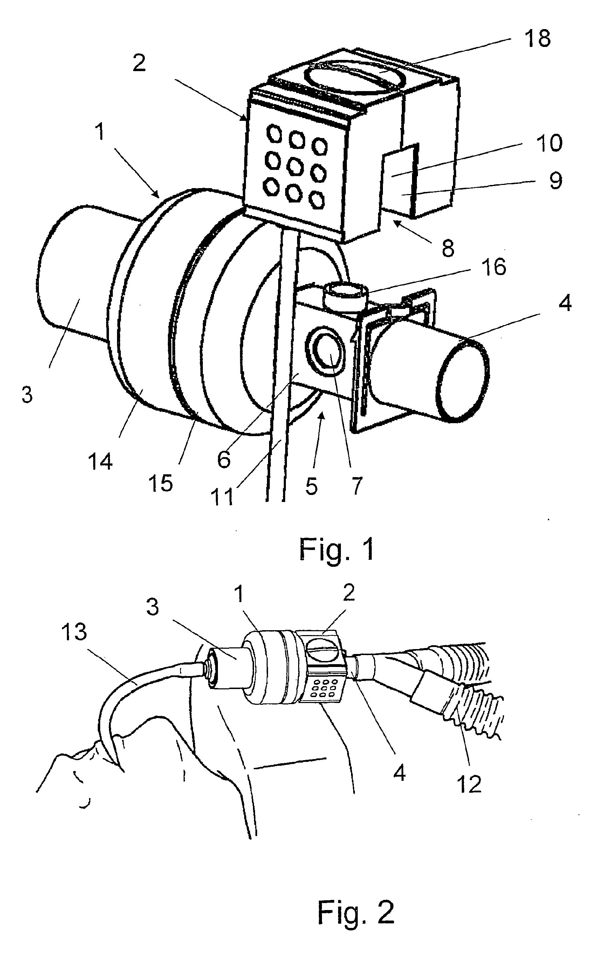

Thus, Fig. 1 shows a gas analyser constructed in accordance with the invention and comprising an adapter 1 and an associated measuring head 2. The adapter 1 has essentially the form of an elongate tube made, for instance, of a plastic material. The adapter 1 has at one end a connector 3 for a hose that leads to the patient. The other end of the adapter carries a connector 4 for a respirator or the like. Located between the two connectors 3, 4 on the adapter 1 is a central portion 5 which is designed to accommodate the measuring head. To this end, the central portion 5 includes two mutually opposing planar surfaces 6, each of which includes a respective window 7 comprised of transparent film.

The measuring head 2 includes a central aperture 8 which extends from one side of the measuring head so as to enable the measuring head to be pushed over the central portion 5 of the adapter. To this end, the aperture is provided with two mutually opposing, generally planar and mutual parallel surfaces 9 that face inwardly towards the aperture. Respective planar surfaces 9 on the measuring head 2 are provided with a light transmitter and a light receiver 10 for transmitting and receiving infrared light respectively. The light transmitter and light receiver are connected by a signal cable 11 to a measuring instrument that analyses the signals obtained from the receiver. The planar surfaces 9 on the measuring head 2 and the planar sides 6 of the central portion 5 of the adapter 1 are mutually designed and dimensioned so that the measuring instrument 2 will be positioned precisely when mounted on the adapter 1, so that light emitted by the light transmitter 10 is able to pass through the central portion 5 of the adapter and through its window 7, and reach the light receiver without being influenced by anything other than that which passes through the interior of the central portion 5 of the adapter.

As will be seen from Fig. 1, the adapter 1 also includes a passive respiratory humidifier or breath moistener 14 between its central portion 5 containing the planar sides 6 for receiving the measuring head and the windows 7 on the planar surfaces, and the connection 3 for connecting the adapter to the patient hose. This passive humidifier may be a so-called HCH, Hygroscopic Condensation Humidifier, or an HME, Heat Moisture Exchanger, of the types generally used in respiratory care. These devices moisturise the respiratory gases by capturing moisture, and to some extent also heat, as the patient breathes, and then return the moisture to the inspiration air as the patient breathes in. Because the passive respiratory humidifier 14 is situated between the patient hose connection 3 and the central portion 5 of the adapter, the expiration gases will be dehumidified when entering the central portion, where the windows 7 are situated, therewith preventing the occurrence of condensation on said windows and also enabling the expiration gas flowing through said central portion 5 to be analysed in a known manner with the aid of the measuring head 2. The passive humidifier 14 is placed in the adapter in the form of a piece of wadding or a roll impregnated with a hygroscopic salt and inserted through the open end of the connector 3.

In addition to the humidifier 14, the adapter 1 may also include bacteria filter 15 situated between the humidifier 14 and the central portion 5. The filter 15 enables bacteria to be removed from the expiration gas, so that, e.g., the oxygen gas concentration can be measured with the aid of a fuel cell without danger of cross contamination between different patients.

As indicated above, a fuel cell can be used in the central portion 5 of the adapter for measuring the oxygen gas concentration of the expiration gas. To this end, a connection 16 to which a fuel cell can be connected may be provided in a side wall of the central portion 5 that lacks a window 7.

Fig. 2 illustrates a patient connected to a respirator with the aid of an arrangement according to the invention. The figure shows that respiratory hoses 12 are connected to the adapter connection 4, and that a patient hose 13 is connected to the patient from the adapter connection 3.

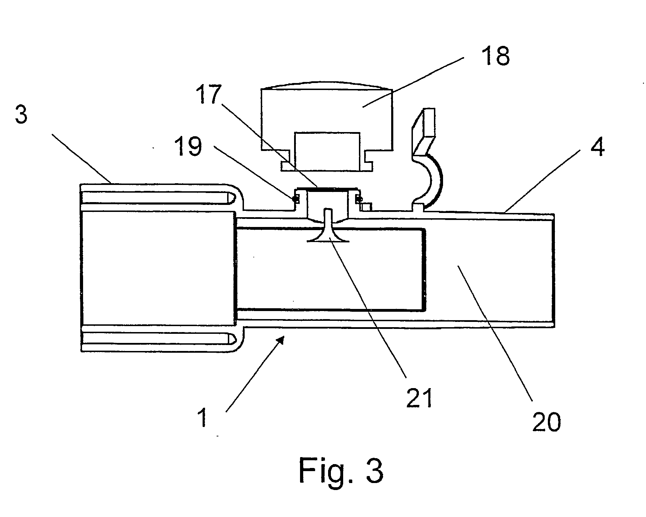

Fig. 3 shows how a fuel cell 18 provided with O-rings 19 can be fastened to the central portion 5 of an adapter. Also shown in the figure is the internal channel 20 in the central portion 5 through which the respiratory gases flow to and from the patient. The internal channel may be provided conveniently with a flow directing means 21 for guiding part of the respiratory gases towards the fuel cell 18 and thereby reducing the step response of the oxygen gas measuring process.

As an alternative to the bacterial filter in the main flow of the adapter 1 as described above, the connection 16 may be provided with a separate bacterial filter 17, for instance in the form of a membrane, as a protection against cross-contamination.

As a further prevention against cross-contamination, a bacteria filter may be arranged in both the main flow, between the patient connection 3 and the central portion 5 of the adapter, and also in the fuel cell connection 16.

The inventive adapter may conveniently be injection-moulded from plastic material and therewith be produced for one-time use at a relatively low cost. The measuring head casing may also be produced from a plastic material although not for one-time use, since the measuring head is used together with the measuring instrument and is not affected or contaminated by the respiratory gases.

## Claims

1. An arrangement for the quantitative analysis of respiratory gases to and from a patient connected to a respirator for breathing assistance, wherein the arrangement includes an adapter (1) that has a connector (4) for connection to a respirator or the like, and a connector (3) for connection to a hose (13) leading to the patient, and wherein the adapter (1) includes connections (5, 16) for a gas analyser measuring head (2) and for a fuel cell (18) which thereby can be located between the respirator connector (4) and the connector (3) for connecting the hoses leading to the patient; and in that the adapter (1) also includes a bacterial filter (15; 17) for protecting the fuel cell (18) from bacteria in the respiratory gases.

2. An arrangement according to Claim 1, **characterised in that** the central portion (5) of the adapter includes a connection (16) for a fuel cell (18) for measuring the oxygen gas content of the respiratory gases.

3. An arrangement according to Claim 1 or 2, **characterised in that** the bacteria filter (15) is arranged in the adapter (1) between the connectors (3) for connection of the hoses to the patient and said central portion (5).

4. An arrangement according to Claim 2, **characterised in that** the bacteria filter (17) is arranged at the fuel cell connection (16).

5. An arrangement according to any one of the preceding Claims, **characterised in that** the adapter (1) includes a flow directing means (21) for guiding part of the respiratory gases to the fuel cell (18).

6. An arrangement according to any one of the preceding Claims, **characterised in that** the adapter includes a passive respiratory gas humidifier (14) between the respirator connector (4) and the connectors for connecting said hoses to the patient.

7. An arrangement according to any one of the preceding Claims, **characterised in that** the measuring head connection includes two windows (7) through which light rays from the measuring head (2) can pass.

## Patentansprüche

1. Vorrichtung für die quantitative Analyse von Atemgasen, welche zu und von einem Patienten strömen, welcher mit einem Beatmungsgerät zur Unterstützung der Bcatmung verbunden ist, wobei die Vorrichtung einem Adapter (1) aufweist, welcher einen Verbinder (4) zur Verbindung mit einem Beatmungsgerät oder dergleichen aufweist, und mit einem Verbinder (3) zur Verbindung mit einem Schlauch (13), welcher zu dem Patienten führt, und wobei der Adapter (1) Verbindungen (5, 16) für einen Messkopf (2) für einen Gasanalysator und für eine Brennstoffzelle (18) aufweist, welche hierdurch zwischen dem Verbinder (4) für das Beatmungsgerät und dem Verbinder (3) für die Verbindung der Schläuche, welche zu dem Patienten führen, angeordnet werden kann, und wobei der Adapter (1) auch einen Bakterienfilter (15, 17) zum Schützen der Brennstoffzelle (18) von Bakterien in den Beatmungsgasen aufweist.

2. Vorrichtung nach Anspruch 1, **dadurch gekennzeichnet, dass** der Zentralabschnitt (5) des Adapters eine Verbindung (16) für eine Brennstoffzelle (18) zum Messen des Sauerstoffgasgohaltes des Beatmungsgases enthält.

3. Vorrichtung nach Anspruch 1 oder 2, **dadurch gekennzeichnet, dass** der Bakterienfilter (15) in dem Adapter (1) zwischen den Verbindern (3) zur Verbindung der Schläuche mit dem Patienten und dem Zentralabschnitt (5) angeordnet ist.

4. Anordnung nach Anspruch 2, **dadurch gekennzeichnet, dass** der Bakterienfilter (17) an der Verbindung (16) der Brennstoffzelle angeordnet ist.

5. Anordnung nach einem der vorhergehenden Ansprüche, **dadurch gekennzeichnet, dass** der Adapter (1) eine Strömungsorientierungseinrichtung (21) zum Leiten eines Teils der Beatmungsgase zu der Brennstoffzelle (18) ausweist.

6. Vorrichtung nach einem der vorhergehenden Ansprüche, **dadurch gekennzeichnet, dass** der Adapter einen passiven Befeuchter (14) für Beatmungsgas zwischen dem Verbinder (4) für das Beatmungsgerät und den Verbindern zur Verbindung der Schläuche mit dem Patienten aufweist.

7. Vorrichtung nach einem der vorhergehenden Ansprüche, **dadurch gekennzeichnet, dass** die Verbindung des Messkopfes zwei Fenster (7) aufweist, durch welche Lichtstrahlen von dem Messkopf (2) bindurch gehen können.

## Revendications

1. Dispositif destiné à l'analyse quantitative de gaz respiratoires acheminés vers un patient et provenant de celui-ci, raccordé à un respirateur pour assistance respiratoire, comprenant un adaptateur (1) ayant un raccord (4) à brancher à un respirateur ou analogue et un raccord (3) à brancher à un tuyau (13) relié au patient, et dans lequel
l'adaptateur (1) comprend des liaisons (5, 16) destinées à une tête de mesure (2) d'analyseur de gaz et à une cellule électrochimique (18) qui peut être de cette manière placée entre le raccord (4) du respirateur et le raccord (3) de branchement des tuyaux reliés au patient, et
l'adaptateur (1) comprend également un filtre bactérien (15 ; 17) pour protéger la cellule électrochimique (18) des bactéries présentes dans les gaz respiratoires.

2. Dispositif selon la revendication **1,**
**caractérisé en ce que**
la partie centrale (5) de l'adaptateur comprend une liaison (16) destinée à une cellule électrochimique (18) servant à mesurer la teneur en oxygène des gaz respiratoires.

3. Dispositif selon la revendication 1 ou 2,
**caractérisé en ce que**
le filtre bactérien (15) dans l'adaptateur (1) est monté entre le raccord (3) de branchement des tuyaux reliés au patient et la partie centrale (5).

4. Dispositif selon la revendication 2,
**caractérisé en ce que**
le filtre bactérien (17) est sur la liaison (16) de la cellule électrochimique.

5. Dispositif selon l'une quelconque des revendications précédentes,
**caractérisé en ce que**
l'adaptateur (1) comprend un moyen (21) dirigeant l'écoulement pour guider une partie des gaz respiratoires vers la cellule électrochimique (18).

6. Dispositif selon l'une quelconque des revendications précédentes,
**caractérisé en ce que**
l'adaptateur comprend un humidificateur passif (14) de gaz respiratoires entre le raccord (4) du respirateur et les raccords de branchement des tuyaux au patient.

7. Dispositif selon l'une quelconque des revendications précédentes,
**caractérisé en ce que**
la liaison de la tête de mesure comprend deux fenêtres (7) à travers lesquelles peuvent passer des rayons lumineux provenant de la tête de mesure (2).
